# EUROPEAN PATENT SPECIFICATION

(11) **EP 1 836 955 B1**
(45) Date of publication and mention of the grant of the patent: **03.12.2008**
(21) Application number: 07251177.7
(22) Date of filing: 20.03.2007
(51) Int. Cl.: A61B 5/05

(54) **Probe assembly**
Sondenanordnung
Ensemble de sonde

(30) Priority: 23.03.2006 GB 0605800
(43) Date of publication of application: 26.09.2007
(73) Proprietor: Depuy International Limited, Beeston, Leeds LS11 8DT (GB); Biosense Webster, Diamond Bar CA 91765 (US); DePuy Orthopädie GmbH, 85551 Heimstetten (DE)
(72) Inventor: Baldewein, Jury c/o Depuy I-Orthopaedics, 85551 Heimstetten (DE); Revie, Ian, Boroughbridge, YO51 9AX (GB); Nitzan, Yaacov c/o Biosense Webster (Israel) Ltd., 39120 Tirat Hacarmel (IL); Brooks, James c/o Depuy International Limited, Leeds LS11 0EA (GB)
(74) Representative: Alton, Andrew

(56) References cited:
- EP-A- 1 570 782
- WO-A-01/30257
- WO-A-94/13200
- WO-A-20/05084572
- US-A1- 2002 143 317
- US-A1- 2003 004 411
- US-B1- 6 332 089

## Description

This invention relates to a probe assembly for use in a surgical procedure. The assembly includes a sensor which can be implanted in a patient to provide information which is useful during or after the surgical procedure.

Sensors can be implanted in a patient for applications such as measuring temperature and analysing material composition. It can be particularly useful in many surgical procedures to use a sensor as a marker to provide location information. The sensor might then be tracked by a tracking system. A tracking sensor of this kind can be used in a catheter while it is navigated through a patient's vessels. It can be used in orthopaedic procedures in which the positions of instruments and implants relative to a patient's bone tissue are monitored.

An implantable sensor can have a cable attached to it. The cable can be used to take signals from the sensor. The signals can be transmitted to an external device through the cable, for example to a data processor. Alternatively, the signal can be transmitted through the cable to a transmitter, from which signals can then be transmitted wirelessly to a receiver.

A sensor which is used to provide location information can make use of electromagnetic signals. US-5391199 and US-5443489 disclose details of systems which are applicable to this aspect of the present invention, in which the coordinates of a probe are determined using one or more field transducers, such as Hall effect devices, coils or other antennae carried on the probe. The sensor can include a coil, which can generate signals in response to externally-applied magnetic fields. The magnetic fields are generally by magnetic field transducers, such as radiator coils, fixed to an external reference frame in known locations. Systems which are concerned with tracking a sensor in 3-dimensional space are also disclosed in WO-96/05768, US-6690963 and US-A-2002/0065455.

US-A-2005/0245821 discloses magnetic tracking systems for generating locating information about objections which are used in a surgical procedure, including instruments and implants. The disclosed systems include one or more location pads which can be attached to the patient, and one or more position transducers which can be implanted in the patient's body. In some embodiments, the location pads transmit magnetic fields which are received by the transducers, in other embodiments, the transducers inside the body transmit magnetic fields which are received by the location pads. In each case, the received field amplitudes are used to determine the coordinates of the transducers in the body relative to one or more of the location pads.

Typically, each location pad is attached to the body surface close to the area in which the position transducer is located. As a result, accurate coordinates may be determined while transmitting relatively weak magnetic fields, and interference with the tracking system from close metal objects is reduced. There is no limitation on movement of the patient's body during the surgical procedure because the location pad moves together with the body.

The specification of International patent application no. PCT/GB2006/000614 discloses detailed of an instrument which can be used to implant a sensor at a desired implantation site in the surgical procedure. The sensor has at least one cord extending from it which can be connected to an external device. The instrument comprises a guide sheath which can defined a path to the implantation site through overlying soft tissue. The sheath has a bore extending along its length between first and second open ends, and a slot in its wall which extends along its length between the first and second open ends.

The sensor can be fastened to the end of a delivery sheath for implantation. The delivery sheath has a bore extending along its length between a first open end where the sensor can be mounted, and an opposite second end. The cord can extend from the sensor along the bore. This sheath has a slot in its side wall which extends along its length between the first and second ends. The delivery sheath can be located within the guide sheath by sliding the delivery sheath in the bore of the guide sheath. The slots of the guide and delivery sheaths can be aligned to allow the cord to be removed from the bores other than at the ends of the bores. Document US 2003/004411 discloses a system as described in the preamble of claim 1.

The instrument which is disclosed in the document referred to above can provide a convenient way to deliver a sensor to an implantation site which is located below layers of overlying tissue, most notably in a bone. It can provide a convenient way to accommodate the cord which is fastened to the sensor.

The present invention provides an assembly which includes an implantable sensor, in combination with an insertion tool and associated electronic circuitry, in which the electronic circuitry and insertion tool can be fitted together to form a convenient assembly.

Accordingly, in one aspect, the invention provides a probe assembly for use in a surgical procedure, which comprises:
a. a sensor which can be implanted in a patient and which is responsive to its local environment for generating data,
b. an insertion tool for the sensor, which can be used to manipulate the sensor and to deliver it to the desired implantation site,
c. electronic circuitry configured to handle a signal which can interact with the sensor,
in which the electronic circuitry and the insertion tool have mating formations by which they can be assembled together.

The invention is as disclosed in the appended set of claims.

The probe assembly of the present invention has the advantage that manipulation of an implantable sensor, which is connected by means of one or more cords, directly or indirectly, to electronic circuitry, is greatly facilitated. In particular, the insertion tool which is used to manipulate the sensor can carry the electronic circuitry so that they can be manipulated as a single unit, especially using one hand. This can represent a significant advantage compared with known arrangements in which a sensor and circuitry might have to be manipulated separately during a procedure as a result of them being connected loosely by means of one or more cords.

The electronic circuitry can handle signals received from the sensor and/or can handle signals sent to the sensor. The signals can be electrical signals, electromagnetic signals or magnetic signals. The electronic circuitry can include circuitry for signal processing and/or signal conditioning.

Preferably, the sensor is responsive to external electromagnetic fields for generating position data.

The nature of the formations by which the electronic circuit and the insertion tool can be assembled together can be selected according to the requirements for a particular application and the materials which are used for the electronic circuit and the insertion tool. Particularly when the material of at least one of electronic circuit and the insertion tool is flexible, it can be appropriate for the mating formations to be provided in the form of a latch, with cooperating ridge and recess. Resilience in the material of the electronic circuitry or the insertion tool can be used to accommodate displacement of one of the ridge and the recess so that the latch can be released to allow separation of the electronic circuitry and the insertion tool.

It can be important for an implanted sensor to be located securely at the relevant implantation site. It can also be important to be able to remove an implanted sensor when its use in the surgical procedure has been completed. A sensor which is particularly suitable for implantation in a patient's bone is disclosed in International patent application number PCT/GB2006/000600. The document includes a sensor which is contained within a jacket. The jacket has a side wall which can be deformed inwardly. The jacket can engage the internal wall of a bore in the bone tissue. The jacket protects the sensor against inward compression forces. Such a sensor is therefore capable of being located securely in a bore in bone tissue. The sensor can be removed from the bore in the bone tissue using a cord which is fastened to the jacket.

The specification of International patent application no. PCT/GB2006/000614 discloses detailed of an instrument which can be used to implant a sensor at a desired implantation site in the surgical procedure. The sensor has at least one cord extending from it which can be connected to an external device. The instrument comprises a guide sheath which can defined a path to the implantation site through overlying soft tissue. The sheath has a bore extending along its length between first and second open ends, and a slot in its wall which extends along its length between the first and second open ends.

The sensor can be fastened to the end of a delivery sheath for implantation. The delivery sheath has a bore extending along its length between a first open end where the sensor can be mounted, and an opposite second end. The cord can extend from the sensor along the bore. This sheath has a slot in its side wall which extends along its length between the first and second ends. The delivery sheath can be located within the guide sheath by sliding the delivery sheath in the bore of the guide sheath. The slots of the guide and delivery sheaths can be aligned to allow the cord to be removed from the bores other than at the ends of the bores.

The instrument which is disclosed in the document referred to above can provide a convenient way to deliver a sensor to an implantation site which is located below layers of overlying tissue, most notably in a bone. It can provide a convenient way to accommodate the cord which is fastened to the sensor.

Preferably, the assembly includes a cord which is connected to the electronic circuitry, though which power can be supplied to the electronic circuitry.

Preferably, the facing surfaces of the electronic circuitry and the insertion tool define a path for the cord so that it can fit between the two when they are assembled together. This can help to control movement of the cord when the assembly is being manipulated during a surgical procedure.

Preferably, the sensor which is for implantation has a signal cable attached to it. The cord can be used to take signals from the sensor. The signals can be transmitted to an external device through the cord, for example to a data processor. Alternatively, the signal can be transmitted through the cable to a transmitter, from which signals can then be transmitted wirelessly to a receiver.

Preferably, the assembly includes a handle by which the single cable can be gripped to apply force to the signal cable through removal of the sensor from an implantation site. It can be preferred for the handle to include a force indicator, which can provide an indication as to the force which is applied to the sensor through the cord. Preferably, the force indicator provides an indication to the user when the force applied to the cable exceeds a predetermined force. Details of a preferred construction of handle are disclosed in the patent application which is being filed in the present application, entitled A Sensor Assembly, bearing agents' ref P211294.

It is particularly preferred that the handle and at least one of the electronic circuitry and the insertion tool have mating formations by which the handled can be assembled with the assembly of the electronic circuitry and the insertion tool.

Preferably, the electronic circuitry is a field generator for generating an electromagnetic field for the sensor.

The insertion tool preferably comprises an elongate shaft, which can have the sensor fitted to it at one end. The shaft can have a bore extending along its length. The shaft and the handle can have a slot, through which the cord can be moved into and out of the bore in the shaft other than through the ends of the bore.

The probe assembly can also include a guide sheath. The guide sheath has a bore extending along its length. The guide sheath has a slot in its side wall extending between its open ends. The bore in the guide sheath is sized so that the insertion tool can be received within it. The guide sheath can have a handle part. The insertion tool can have a handle part. The handle parts on the guide sheath and the insertion tool can fit together to form an assembly.

An instrument which comprises a guide sheath and an insertion tool having these features is disclosed in International patent application number PCT/GB2006/000614.

Embodiments of the present invention will now be described by way of example with reference to the accompanying drawings in which:
Figure 1 shows a sensor assembly comprising an implantable sensor and an associated electronic circuit.
Figure 2 shows a side view of an instrument according to the invention assembled with a sensor;
Figure 3a shows a perspective view of the delivery sheath of the instrument shown in Figure 2 with a drill bit partially inserted therethrough;
Figure 3b shows the delivery sheath shown in Figure 3a with the drill bit fully received within the guide sheath;
Figure 4 shows a side view of the instrument shown in Figure 2 with the guide sheath and delivery sheath separated;
Figure 5 is an exploded view of the field generator component of the probe assembly of the invention.
Figure 6 is a view from below of the probe assembly of the invention, with the insertion tool separated from the field generator.
Figure 7 is a view from below of the assembly of the invention with the field generator and insertion tool assembled together.

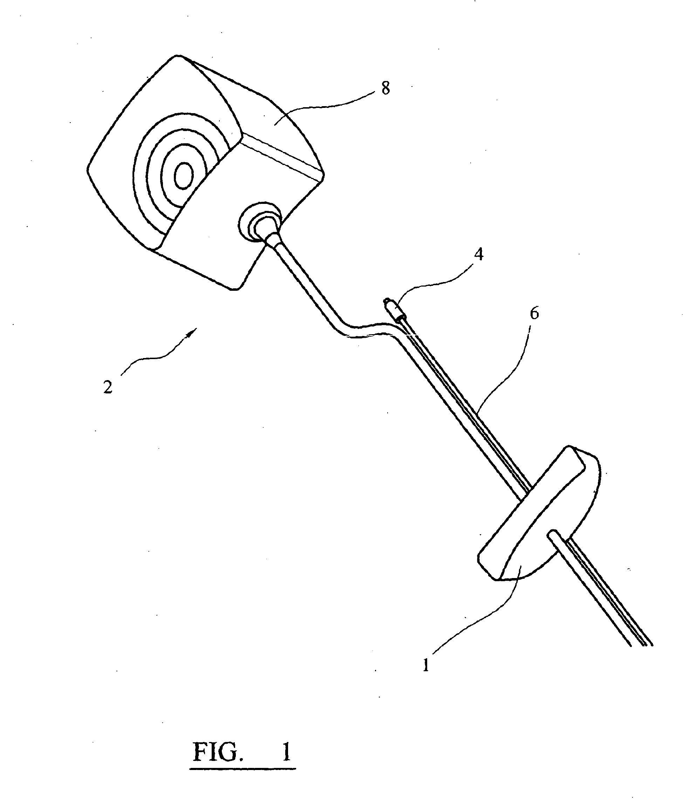

Referring to the drawings, Figure 1 shows a sensor assembly 2 which can be used in a surgical procedure. The assembly comprises an implantable sensor 4, which has a signal cable 6 extending from it. The sensor assembly also includes electronic circuitry 8 in the form of a field generator. The field generator can generate an electro-magnetic field for the sensor. The sensor is responsive to the electro-magnetic field in its local environment. The sensor 4 is able to generate position data in response to the externally applied electromagnetic field from the field generator 8. Sensor assemblies of the kind shown in Figure 1, which include a transducer sensor and a location pad field generator are known from commercial products, for example as sold by DePuy International Limited and related companies. Information about such assemblies their uses is disclosed in US-A-2005/0245821 and International patent application no. PCT/GB2006/000614.

In use in an orthopaedic surgical procedure, the sensor 4 is implanted in a bore in a bone using an implantation tool, with the cable 6 extending from the sensor and through a channel in the overlying soft tissue. The field generator 8 is located on the surface of the patient tissue, close to the area in which the sensor is implanted. As a result, accurate coordinates may be determined while transmitting relatively weak magnetic fields, and interference with the tracking system from close metal objects is reduced. There is no limitation on movement of the patient's body during the surgical procedure because the field generator moves together with the body.

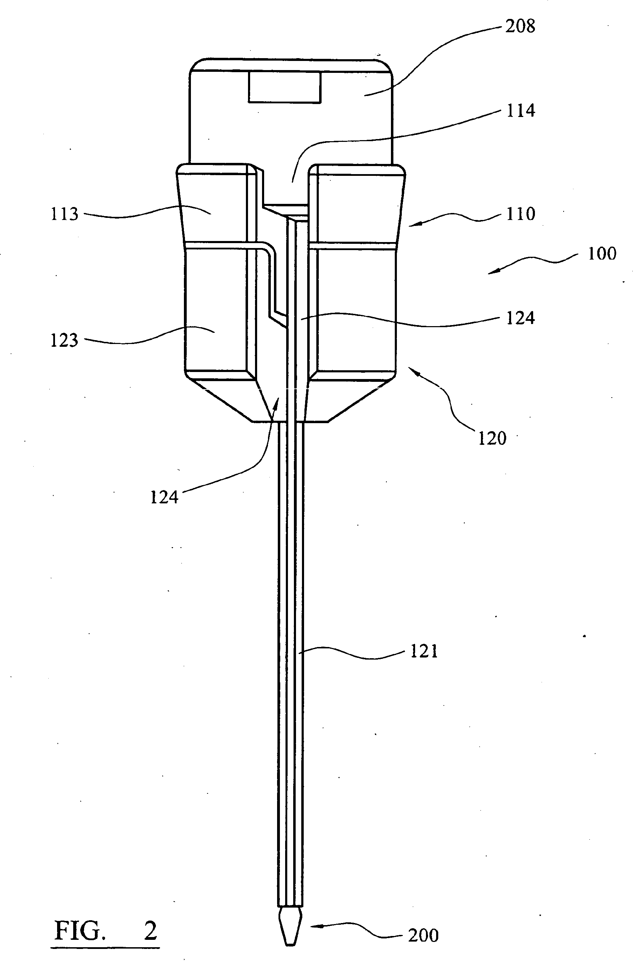

The assembly of the invention includes an insertion tool for the sensor. The insertion tool can be used with a guide sheath. A preferred insertion tool is in the form of a delivery sheath. An insertion tool 100 which comprises a delivery sheath 110 and a guide sheath 120 is shown in Figures 2 to 5. The guide sheath 120 comprises a tubular sheath 121 having a first end 128 which during use abuts the surface of the bone, and a second end 129 distal to the first end. The tubular sheath 121 has a bore extending through it, along its length, between the first 128 and second 129 ends. The tubular sheath 121 has a slot 122 formed in it that extends along its length, between the first 128 and second 129 ends, so as to provide a path between the bore of the tubular sheath and the exterior of the tubular sheath.

The tubular sheath 121 has at its first end 128 a plurality of teeth 127 extending axially therefrom for engagement with the bone at the site in which the sensor 200 is to be inserted. The teeth 127 are mutually spaced around the first end 128 of the tubular sheath 121.

The guide sheath 120 includes a substantially annular handle 123 to facilitate holding of the guide sheath by a surgeon. The handle 123 extends around the circumference of the tubular sheath 121 at the second end 129. The handle 123 has a slot 124 extending through its substantially annular side wall 125, the slot of the handle being aligned with the slot 122 of the tubular sheath 121 so as to provide a path between the exterior of the side wall 125 and the bore extending through the tubular sheath 121.

The substantially annular side wall 125 of the handle 123 extends axially away from the first end 128 of the tubular sheath and as such defines a socket 126 in the handle 123 for receiving a spigot 116 of the handle 113 of the delivery sheath 110 described hereinafter. The cross-sectional shape of the socket 126 taken in a plane perpendicular to the longitudinal axis of the tubular sheath 121 is generally that of a circle having a flat side.
As described hereinafter, this ensures that the spigot 116 of the delivery sheath 110 can only be received in the socket 126 in one angular orientation.

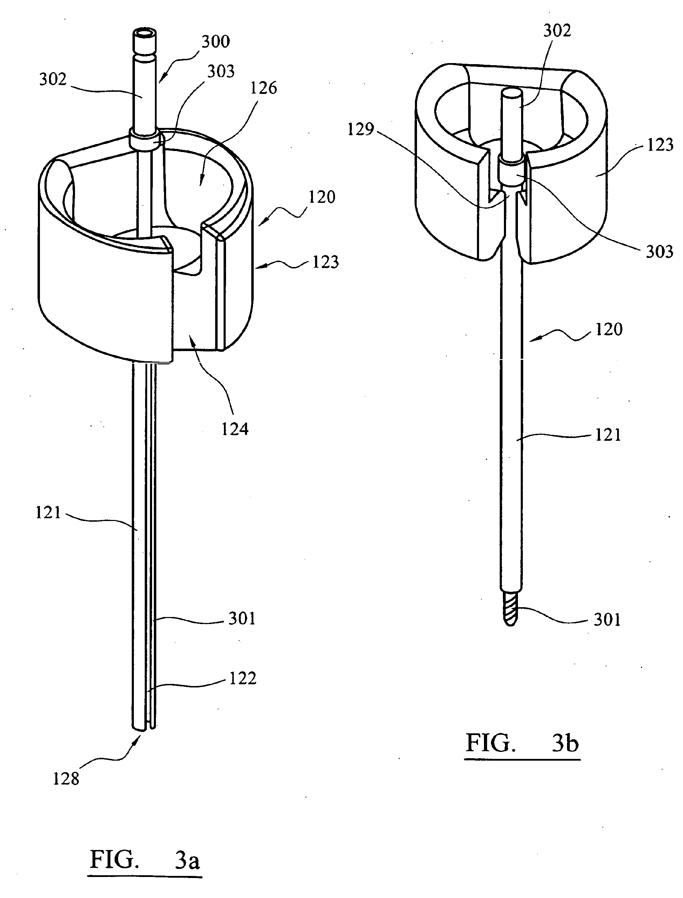

The tubular sheath 121 is configured to be able to receive a drill bit 300 through its bore. As shown in Figures 3a and 3b, the drill bit 300 comprises a cutting end 301 at a first end of the drill bit for cutting a hole into a bone, and a second end 302 distal to the cutting end which is configured to facilitate attachment of the drill bit to a tool (not shown) for imparting a rotational force on the drill bit. An annular flange 303 is provided towards the second end of the drill bit. The diameter of the annular flange 303 is greater than the diameter of the bore within the tubular sheath 121, to limit the extend by which the drill bit 300 can slide through the tubular sheath 121. The flange 303 thereby controls the extent by which the drill bits 300 can extend through the tubular sheath and accordingly can control the depth of the hole created by the drill bit 300.

The delivery sheath 110 comprises a tubular sheath 111 that has a first end 119 which during use is proximal the surface of the bone, and a second end 130 distal to the first end. The tubular sheath 111 has a bore extending through it, along its length, between the first 119 and second 130 ends. The tubular sheath 111 has a slot 112 formed in it that extends along its length, between the first 119 and second 130 ends, so as to provide a path between the bore of the tubular sheath and the exterior of the tubular sheath. The tubular sheath 111 of the delivery sheath 110 is shaped and sized so as to be able to slide within the bore of the tubular sheath 121 of the guide sheath 120, and described in more detail hereinafter.

The delivery sheath 110 includes a substantially annular handle 113 to facilitate holding of the delivery sheath by a surgeon. The handle 113 extends around the circumference of the tubular sheath 111 at its second end 130. The handle 113 has a slot 114 extending through its substantially annular side wall 115, the slot of the handle being aligned with the slot 112 of the tubular sheath 111 so to provide a path between the bore of the tubular sheath 111 and the exterior of the handle 113.

The handle 113 includes a spigot 116 that extends axially towards the first end 119 of the tubular sheath 111. The socket 126 of the handle 123 of the guide sheath 120, and the spigot 116 are shaped and sized so as to enable the spigot to be received in the socket.

The substantially annular side wall 115 extends away from the first end 119 of the tubular sheath 111 and defines a socket 117 in the handle 113 for receiving an external device such as the reference pad 208 which is connected to the sensor 200 via a cable 206.

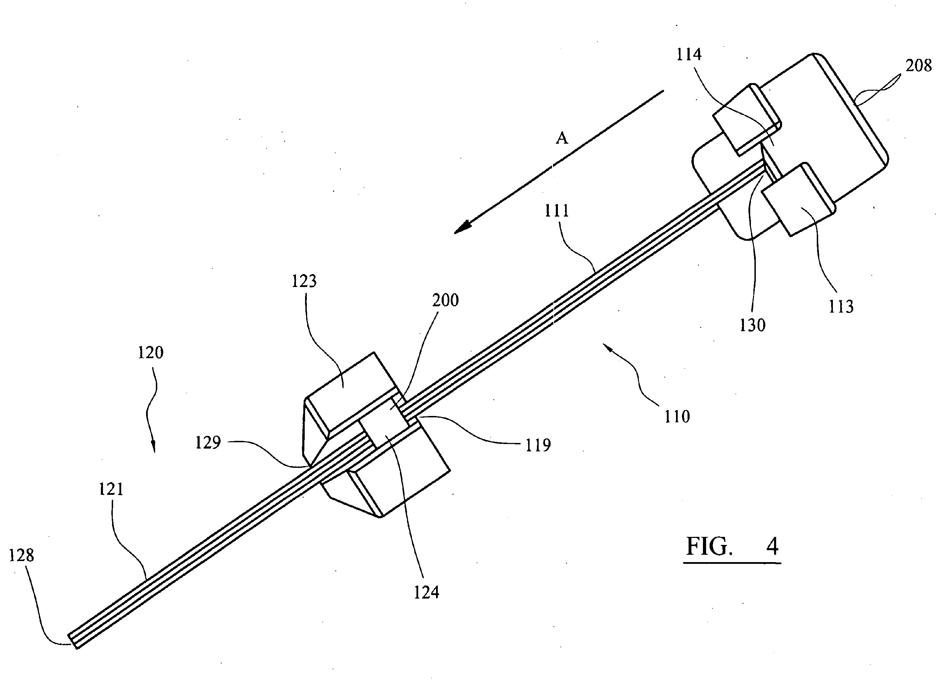

As shown in Figure 4, the delivery sheath 110 can be slidingly received within the guide sheath 120. As described above, the tubular sheath 111 of the delivery sheath 110 is shaped and sized so as to be able to slide within the tubular sheath 121 of the guide sheath 120, and the spigot 116 of the delivery sheath 110 is shaped and sized so that it can be received within the socket 126 of handle 123 the guide sheath 120. The spigot 116 and socket 126 are shaped so that the spigot can be received in the socket in one angular orientation only. The spigot 116 and socket 126 are configured so that when the spigot 116 is properly received within the socket 126, the slot 112 and 114 of the delivery sheath 110 are aligned with the slots 122 and 124 of the guide sheath. Therefore, as shown in Figure 2, when the delivery sheath 110 is fully received within the guide sheath 120, the slots 112, 114, 122, 144 provide a path between the bore of the tubular sheath 111 of the delivery sheath 110 and the exterior of the assembled instrument.

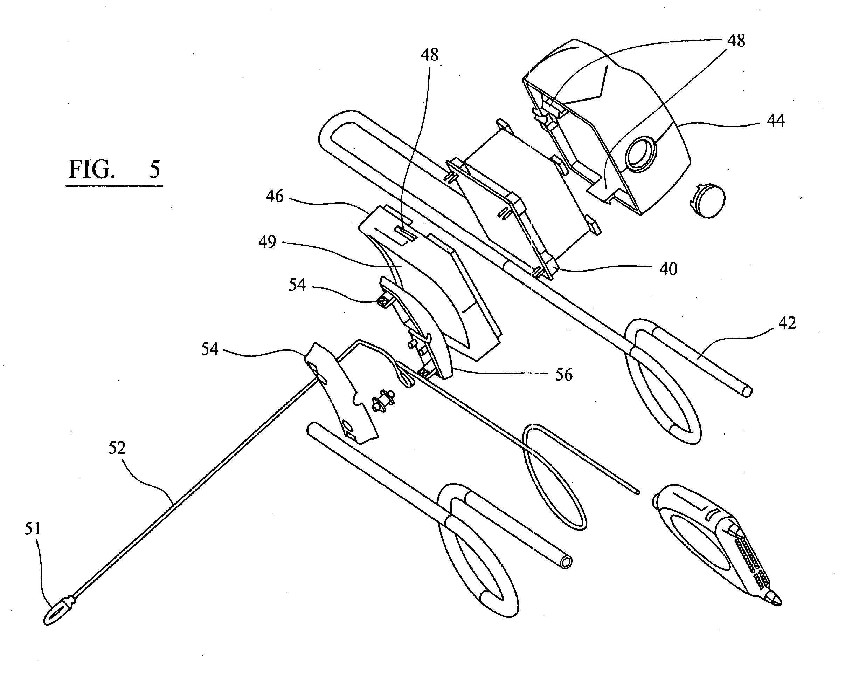

Figure 5 shows the field generator component of the assembly. The field generator component includes hardware 40 for generating the magnetic field. Such components as used in surgical procedures are known. The field generator includes a cable 42 through which power is supplied to the field generator hardware. The field generator hardware 40 is housed in a two part case 44, 46. The two parts of the case are capable of being fitted together by means of cooperating latches and detents 48. The lower part 46 of the case has a concave rounded lower surface 49 so that it is appropriate profiled to be fitted against the surface of a patient's limb. The component which is shown in Figure 5 is therefore suitable for use in a surgical procedure on a patient's limb. A flat lower surface might be appropriate when the component is intended for use in a procedure which is performed on a patient's thoracic cavity or spine.

The lower surface 49 of the lower part of the case for the field generator hardware can have fitted to it a quantity of a part of a hook and loop material (such as either the hook part of the loop part of the material sold under the trade mark Velcro) which can be used to fasten the case to a patient body.

Figure 5 also shows the sensor component 50, of the assembly, with a cord 52 extending from it. The assembly includes a handle 54 which can be used to grip the cord 52 so as to apply a pulling force to the cord. The handle has a central passageway 54 extending through it, through which the cord 52, together with the cable 42 for the field generator hardware, can pass. The engagement of the cord with the handle can be as described in the patent application which is being filed in the present application, entitled A Sensor Assembly, bearing agents' ref P211294. The shape of the handle is arranged so that the handle is a snug fit against the lower surface 49 of the lower part of the case. Accordingly, the handle has a convex upper surface 56.

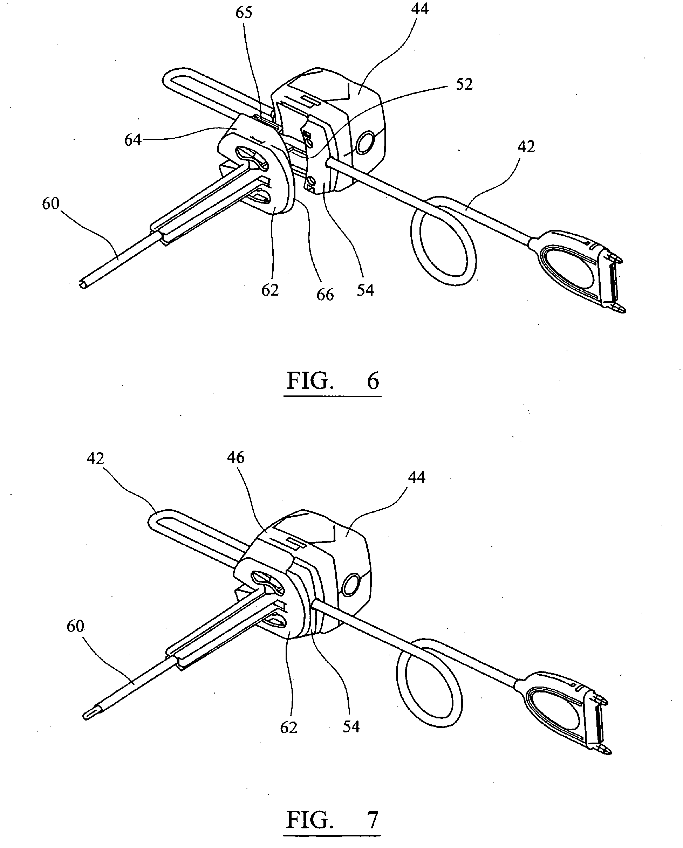

Figure 6 shows the handle 54 fitted against the underside of the lower part 46 of the case for the field generator hardware, with the cable 42 for the field generator hardware extending along the lower surface of the case and through the handle.

Figure 6 also shows a insertion tool delivery sheath 60 which can be used to insert the sensor component 50 in a bore in a patient's bone or other tissue. It has a moulded handle part 62 for manipulating the sheath. The handle part 62 is shaped with a raised platform part 64 so that it fits against the underside of the lower part 46 of the case for the field generator hardware, and a lower platform part 66 which fits against the handle 54. The raised platform part 64 provides a pathway to accommodate the cable 42 for the field generator hardware.

The handle part 62 has latch portions 65 which can engage detents on the case for the field generator hardware to retain the two together. The latches can be released by depressing the latch portions.

The handle 54 on the cord 52, the case for the field generator hardeware, and the handle part 62 for the insertion tool are preferably made from polymeric material. The material should be capable of being cleaned by one or more conventional cleaning techniques which are used in relation to surgical instruments, such as irradiation and exposure to water vapour at elevated temperatures and pressures. Examples of suitable materials might include low molecular weight polyolefins.

In other embodiments, the electronic circuitry is not a field generator and the sensor is not, or is not solely, an electromagnetic field sensor used for tracking purposes. More generally, the sensor can be any sensor which is responsive to its local environment and which can generate data. For example, the sensor may measure or determine a property of its local environment and generate data or a signal representative of that local property.
For example, the sensor can be a temperature sensor which can generate an output signal which is representative of the temperature local to the sensor and the electronic circuitry 8 can include signal processing, handling or conditioning circuitry which accepts the sensor output signal as input and generates a processed or modified signal as output, for example a digital signal representative of the local temperature.

As well as, or alternatively to, handling signals received from the sensor, the electronic circuitry can act as a source of electrical signals used by the sensor. For example, if the sensor is an active device which requires a power source, the electronic circuitry 8 can include a power source and can supply electrical power to the sensor. In other embodiments, the electronic circuitry can include, for example, an amplifier or amplifiers, *e.g.,* for amplifying the signal received from the sensor, a filter or filters, *e.g.,* for filtering the signal received from the circuitry, or interface circuitry, *e.g.* for adapting signals to be sent to the sensor or received from the sensor so that they can be used or provided by a secondary device, such as a computer or other data processing hardware.

## Claims

1. A probe assembly for use in a surgical procedure, which comprises:
a. a sensor (50) which can be implanted in a patient and which is responsive to its local environment for generating data,
b. an insertion tool (60) for the sensor (50) having a handle, which can be used to manipulate the sensor and to deliver it to the desired implantation site,
c. electronic circuitry (40) configured to handle a signal which can interact with the sensor,
**characterized in that** the electronic circuitry has a housing (44) not including the sensor and in which the housing and the handle of the insertion tool have mating formations by which they can be assembled together.

2. A probe assembly as claimed in claim 1, in which the electronic circuitry is a field generator for generating an electromagnetic field for the sensor.

3. A probe assembly as claimed in claim 2, which includes a cord which is connected to the electronic circuitry through which power can be supplied to the electronic circuitry.

4. A probe assembly as claimed in claim 3, in which the facing surfaces of the electronic circuitry and the insertion tool define a path for the cord so that it can fit between the two when they are assembled together.

5. A probe assembly as claimed in claim 1, in which the sensor has a signal cable attached to it.

6. A probe assembly as claimed in claim 5, which includes a handle by which the signal cable can be gripped to apply force to the signal cable for removal of the sensor from the implantation site.

7. A probe assembly as claimed in claim 6, in which the handle and at least one of the electronic circuitry and the insertion tool have mating formations by which the handle can be assembled with the assembly of the electronic circuitry and the insertion tool.

8. A probe assembly as claimed in claim 1, in which the face of the electronic circuitry which faces towards the insertion tool carries a quantity of one part of a hook and loop fastener material.

## Patentansprüche

1. Sondenanordnung zur Benutzung bei einem chirurgischen Eingriff, die Folgendes umfasst:
a. einen Sensor (50), der in einem Patienten implantiert werden kann und der auf seine lokale Umgebung zur Erzeugung von Daten anspricht,
b. ein Einsetzhilfsmittel (60) für den Sensor (50) mit einem Handstück, das benutzt werden kann, um den Sensor zu beeinflussen und um ihm an den gewünschten Implantationsort zu führen,
c. eine elektronische Schaltung, die dafür konfiguriert ist, ein Signal zu behandeln, das mit dem Sensor interagieren kann,
**dadurch gekennzeichnet, dass** die elektronische Schaltung ein Gehäuse (44) aufweist, das den Sensor nicht enthält, und dass das Gehäuse und das Handstück des Einsetzhilfsmittels zueinander passende Formen aufweisen, mittels derer diese miteinander zusammengebaut werden können.

2. Sondenanordnung nach Anspruch 1, bei der die elektronische Schaltung eine Feldgenerator zum Erzeugen eines elektromagnetischen Felds für den Sensor ist.

3. Sondenanordnung nach Anspruch 2, die ein Kabel aufweist, das mit der elektronischen Schaltung verbunden ist und durch welches die elektronische Schaltung mit Leistung versorgt werden kann.

4. Sondenanordnung nach Anspruch 3, bei welcher die gegenüberliegenden Seiten der elektronischen Schaltung und des Einsetzhilfsmittels einen Weg für das Kabel definieren, so dass es zwischen den beiden befestigt werden kann, wenn sie zusammengebaut sind.

5. Sondenanordnung nach Anspruch 1, bei welcher der Sensor ein Signalkabel aufweist, das an diesem angebracht ist.

6. Sondenanordnung nach Anspruch 5, die ein Handstück umfasst, mittels dessen das Signalkabel ergriffen werden kann, um eine Kraft auf das Signalkabel zum Entfernen des Sensors von dem Implantationsort anzuwenden.

7. Sondenanordnung nach Anspruch 6, bei welcher das Handstück und die elektronische Schaltung und/oder das Einsetzhilfsmittel zueinander passende Formen aufweisen, mittels derer das Handstück mit dem Aufbau aus der elektronischen Schaltung und dem Einsetzhilfsmittels zusammengebaut werden kann.

8. Sondenanordnung nach Anspruch 1, bei welcher die Seite der elektronischen Schaltung, welche zu dem Einsetzhilfsmittel weist, eine Größe eines Teils eines Befestigungsmaterials mit Haken und Ösen trägt.

## Revendications

1. Ensemble de sonde à utiliser dans une intervention chirurgicale, qui comprend :
a. un capteur (50) qui peut être implanté dans un patient et qui répond à son environnement local pour générer des données,
b. un outil d'insertion (60) du capteur (50) ayant une poignée, qui peut être utilisé pour manipuler le capteur et pour l'acheminer jusqu'au site d'implantation souhaité,
c. un circuit électronique (40) configuré pour traiter un signal qui peut interagir avec le capteur,
**caractérisé en ce que** le circuit électronique a un logement (44) ne comprenant pas le capteur et dans lequel le logement et la poignée de l'outil d'insertion ont des formations d'appariement qui permettent de les assembler l'un à l'autre.

2. Ensemble de sonde selon la revendication 1, dans lequel le circuit électronique est un générateur de champ servant à générer un champ électromagnétique pour le capteur.

3. Ensemble de sonde selon la revendication 2, qui comprend un cordon qui est relié au circuit électrique par lequel de l'électricité peut être fournie au circuit électronique.

4. Ensemble de sonde selon la revendication 3, dans lequel les surfaces se faisant face du circuit électronique et l'outil d'insertion définissent une voie pour le cordon de sorte qu'il puisse s'ajuster entre les deux lorsqu'ils sont assemblés l'un à l'autre.

5. Ensemble de sonde selon la revendication 1, dans lequel le capteur a un câble de signal fixé à celui-ci.

6. Ensemble de sonde selon la revendication 5, qui comprend une poignée par laquelle le câble de signal peut être saisi pour appliquer une force au câble de signal pour retirer le capteur du site d'implantation.

7. Ensemble de sonde selon la revendication 6, dans lequel la poignée et au moins l'un des éléments comprenant le circuit électronique et l'outil d'insertion ont des formations d'appariement permettant à la poignée d'être assemblée avec l'ensemble constitué par le circuit électronique et l'outil d'insertion.

8. Ensemble de sonde selon la revendication 1, dans lequel l'avant du circuit électronique qui fait face à l'outil d'insertion porte une quantité d'une partie d'un crochet et d'un matériau de fixation de boucle.
